# EUROPÄISCHE PATENTSCHRIFT

(11) **EP 1 455 581 B1**
(45) Veröffentlichungstag und Bekanntmachung des Hinweises auf die Patenterteilung: **22.06.2016**
(21) Anmeldenummer: 02804900.5
(22) Anmeldetag: 16.12.2002
(51) Int. Cl.: A01N 49/00, A01P 3/00, C09J 11/06, C09D 5/34

(54) **HEMMUNG DER ASEXUELLEN VERMEHRUNG VON PILZEN**
INHIBITION OF THE ASEXUAL REPRODUCTION OF FUNGI
AGENTS EMPECHANT LA MULTIPLICATION ASEXUEE DE CHAMPIGNONS

(30) Priorität: 18.12.2001 DE 10162142
(43) Veröffentlichungstag der Anmeldung: 15.09.2004
(73) Patentinhaber: Henkel AG & Co. KGaA, 40589 Düsseldorf (DE)
(72) Erfinder: BOCKMÜHL, Dirk, 42113 Wuppertal (DE); BREVES, Roland, 40882 Ratingen (DE); WEIDE, Mirko, 40223 Düsseldorf (DE); HEINZEL, Michael, 53127 Bonn (DE)
(86) Internationale Anmeldenummer: PCT/EP2002/014306
(87) Internationale Veröffentlichungsnummer: WO 2003/051124

(56) Entgegenhaltungen:
- EP-A- 1 059 032
- WO-A-00/27981
- WO-A-01/24769
- WO-A-99/09824
- DE-A- 19 523 320
- V. G. DE BILLERBECK, C. G. ROQUES, J.-M. BESSIÈRE, J.-L. FONVIEILLE & R. DARGENT: "Effects of Cymbopogon nardus (L.) W. Watson essential oil on the growth and morphogenesis of Aspergillus niger." CAN. J. MICROBIOL., Bd. 47, 2001, Seiten 9-17, XP009008006
- T. BELAICHE, A. EL HALOUAT & A. TANTAOUI-ELARAKI: "Etude d'influence des terpenes sur la sporulation d'Aspergillus" IND. ALIMENT. AGRICOL., Bd. 116, 1999, Seiten 27-29, XP009007965
- DATABASE WPI Section Ch, Week 197837 Derwent Publications Ltd., London, GB; Class A60, AN 1978-66181A XP002235743 & JP 53 091123 A (KURARAY CO LTD), 10. August 1978 (1978-08-10)

## Beschreibung

Die Erfindung betrifft Klebstoffe oder Dichtungsmassen, die 0,001 bis 1,5 Gew.-% Farnesol enthalten.

Pilze, insbesondere Schimmelpilze, verursachen erhebliche Probleme im Bereich der Baubiologie, da die von ihnen in die Raumluft abgegebenen Sporen häufig allergieerzeugend sind. Eine Bekämpfung solcher Pilze mit bioziden Wirkstoffen geht mit einem erhöhten Risiko der Resistenzbildung einher, so dass nach einiger Zeit neue antimikrobielle Substanzen gefunden werden müssen, die gegen diese resistent gewordenen Mikroorganismen wirken. Biozide sind außerdem ökologisch und toxikologisch nicht immer unbedenklich. Zu den unerwünschten Wirkungen der Verbreitung von Schimmelpilzen gehören insbesondere Verfärbungen (beispielsweise an Wänden, Fugendichtmassen und anderen Badoberflächen), die durch pigmentierte Sporen hervorgerufen werden.

Immer häufiger werden empfindliche Textilien, wie z. B. Seide oder Mikrofaser, zu Kleidungsstücken verarbeitet, die nur bei 30 oder 40 °C gewaschen werden können. Dadurch werden Pilze, wie beispielsweise die humanpathogene *Candida albicans,* nicht abgetötet. Insbesondere nach einer Pilzinfektion kann es durch solche auf Kleidungsstücken haftenden, nicht abgetöteten Pilze zu einer Reinfektion kommen.

Daher wurden bisher antimikrobielle Substanzen eingesetzt, die entweder das Wachstum der Pilze hemmen (Fungistatika) oder diese abtöten (Fungizide). Häufig werden dazu nicht-selektive antimikrobielle Substanzen eingesetzt, die sowohl gegen Bakterien als auch gegen Pilze wirken. Nachteilig ist daran, dass solche z. B. in Wasch- und Reinigungsmitteln verwendeten Biozide oder Biostatika die Abwässer belasten und somit auch die mikrobiellen Klärstufen in den Kläranlagen in ihrer Funktion beeinträchtigen.

V. G. de Billerbeck et al., "Effects of Cymbopogon nardus (L.) W. Watson essential oil on the growth and morphogenesis of Aspergillus niger," Can. J. Microbiol., Bd. 47, 2001, Seiten 9-17 beschreibt die Wachstums- und Sporenbildungshemmung bei *Aspergillus niger* durch Anwesenheit eines Öls aus *Cymbopogon nardus.* Dieses Öl enthält Terpenverbindungen, wie Citronellal, Geraniol und Citronellol sowie Eugenol, Linalool, Candinen und Limonen.

T. Belaiche et al., "Etude de l'influence des terpenes sur la sporulation d'Aspergillus," Ind. Aliment. Agricol., Bd. 116, 1999, Seiten 27-29 beschreibt die antimykotische Wirkung der Terpenalkohole, Citronellol, Menthol und Terpineol, auf die Sporulation von *Aspergillus flavus, A. parasiticus* und *A. niger.*

DE 195 23 320 A1 betrifft Desinfektionsmittel-Konzentrate, enthaltend neben an sich bekannten, desinfizierend wirkenden Verbindungen und üblichen Hilfsstoffen sowie Wasser ein oder mehrere Terpen(e) in Konzentrationen von 0,1 bis 50%, bezogen auf das Gesamtgewicht des Desinfektionsmittel-Konzentrats.

WO 01/24769 A1 betrifft eine antimikrobielle Zusammensetzung, dadurch gekennzeichnet, dass sie eine wirksame Menge von einem oder mehreren Wirkstoffen enthält, die eine antimikrobiellen Aktivität von 100% aufweisen, gemessen durch den mikrobiellen Reduktionstest.

JP 53 091 123 A betrifft wirksame antibakterielle Mittel, mit geringer Toxizität für den Menschen und mit keiner Reizung der Haut, enthaltend Farnesol als Wirkstoff.

Überraschenderweise wurde gefunden, daß der Einsatz von Farnesol auf oder in durch Pilze befallenen Materialien die Ausbreitung der Pilze zu unterbinden vermochte, ohne jedoch die Pilze abzutöten.

Gegenstand der vorliegenden Erfindung sind daher Klebstoffe oder Dichtungsmassen, die 0,001 bis 1,5 Gew.-% Farnesol enthalten, zur Hemmung der asexuellen Vermehrung von Pilzen.

Erfindungsgemäß umfaßt der Begriff asexuelle Vermehrung insbesondere Sporenbildung, Knospung und Fragmentierung.

Vorteilhafterweise werden die Pilze bei der erfindungsgemäßen Verwendung weder in ihrem Wachstum gehemmt noch abgetötet, es wird lediglich die asexuelle Vermehrung gehemmt bzw. unterdrückt. Der Selektionsdruck für die Bildung von Resistenzen ist daher gering.

Ein weiterer Vorteil der Erfindung ist es, dass Farnesol im Vergleich mit Fungiziden oder Fungistatika bereits in geringen Endkonzentrationen wirksam ist und daher kaum unerwünschte Nebenwirkungen befürchtet werden müssen.

Gemäß einer bevorzugten Ausführungsform der vorliegenden Erfindung wird Farnesol zur Hemmung der Sporenbildung verwendet. Unter Sporenbildung ist dabei sowohl die Bildung von Vermehrungsformen z.B. Konidien, Gonitocysten, Sporangiosporen, Arthrosporen, Blastosporen und deren assoziierten Organen (z.B. Konidiophoren) als auch von Dauerformen (z.B. Chlamydosporen) zu verstehen.

Da Schimmelpilzsporen in der Raumluft ubiquitär vorhanden sind, kann ein Schimmelbefall nicht grundsätzlich verhindert werden. Die Hemmung der Sporulation der auswachsenden Pilzkolonien bietet allerdings die Möglichkeit, das Risiko einer Schimmelpilzallergie erheblich zu verringern und die Verbreitung des Pilzes vollständig aufzuhalten bzw. beträchtlich zu verzögern. Verfärbungen aufgrund von Sporenbildung werden ebenfalls stark vermindert oder vollständig verhindert.

Der Klebstoff oder die Dichtungsmasse der vorliegenden Erfindung kann optional zusätzlich Monoterpene, Sesquiterpene und/oder Diterpene oder deren Derivate ausgewählt unter Ethern von Farnesol, Säuren, wie zum Beispiel Farnesolsäure, sowie deren Estern und anderen funktionelle Gruppen tragenden Monoterpene, Sesqui- bzw. Diterpenen enthalten. Geeignet sind dabei sowohl die trans- als auch die cis-Isomere. Ebenfalls darunterfällt α-Farnesen (3,7,11-Trimethyl-1,3,6,10-Dodekatetraen) sowie β-Farnesen (7,11-Dimethyl-3-Methylen-1,6,10-Dodekatrien) und Nerolidol (3,7,11-Trimethyl-1,6,10-Dodekatrien-3-ol) sowie Bisabolen, Sesquiphellandren, Zingiberen, Cadinen, aryl-Tumeron, Tumeron, Xanthorrhizol, Vulgaren und β-Selinen. Als Monoterpene sind beispielsweise α- bzw. β-Ocimen, Linalool, Linalylacetat, Carene, Terpineole, Nerol, Nerolsäure, Geraniol, Geraniumsäure, α- bzw. β-Phellandren und/oder Thujon, insbesondere Geraniol, Linalool und/oder Thujon bevorzugt geeignet. Als Beispiel für die Diterpene sei hier Geranylgeraniol (3,7,11,15-Tetramethyl-2,6,10,14-Hexadekatetraen-1-ol) sowie seine Isomere und Derivate genannt. Es können ebenfalls bevorzugt Pflanzenextrakte eingesetzt werden, die Mono-, Sesqui- und/oder Diterpene enthalten (beispielsweise Geraniumöl, Rosenöl, Orangenblütenöl, Lavendelöl, Jasminöl, Basilikumöl, Citronellöl, Zypressenöl, Zedernblätteröl, Korianderöl, Rosenholzöl, Pimentöl, Ingweröl oder Nelkenöl). Gemäß einer besonders bevorzugten Ausführungsform sind die optionalen Monoterpene, Sesquiterpene und/oder Diterpene oder deren Derivate ausgewählt unter Farnesolsäure.

Gemäß einer besonderen Ausführungsform werden Farnesol und die optionalen Monoterpene, Sesquiterpene und/oder Diterpene oder deren Derivate in solchen Endkonzentrationen eingesetzt, dass sie nicht fungizid (pilzabtötend) oder fungistatisch (pilzwachstumshemmend) wirken. Ein besonderer Vorteil dieser Ausführungsform ist es, dass das Risiko einer Resistenzbildung gegenüber den verwendeten Stoffen relativ gering ist, da die Pilze weder abgetötet noch ihr Wachstum gehemmt werden. Diese minimalen Hemmkonzentrationen können in dem Fachmann bekannter Weise einfach bestimmt werden.

Gemäß einer weiteren besonderen Ausführungsform kann der Klebstoff oder die Dichtungsmasse der vorliegenden Erfindung die optionalen Monoterpene, Sesquiterpene und/oder Diterpene oder deren Derivate zu 0,000001 bis 3 Gew.-% enthalten. Ein besonderer Vorteil dieser Ausführungsform ist es, dass nur geringe Konzentrationen dieser Stoffe vorhanden sein müssen, damit die asexuelle Fortpflanzung der Pilze vermindert bzw. im wesentlichen ganz verhindert wird. Bevorzugt sind die Monoterpene, Sesquiterpene und/oder Diterpene oder deren Derivate zu 0,00001 bis 1 Gew.-% und insbesondere zu 0,0001 bis 0,5 Gew.-% enthalten. Besonders bevorzugt sind Bereiche zwischen 0,0001 und 0,1 Gew.-%.

Die Konzentrationen, die im Endprodukt zum gewünschten Ergebnis führen, sind bedeutend geringer als die angegebenen, da für viele Produkte Verdünnungen berücksichtigt werden müssen. In der fertigen Anwendungslösung zeigen insbesondere Konzentrationen von 0,0001 bis 1 Gew.-% eine besonders gute adhäsionshemmende Wirkung. Bevorzugt werden 0,001 bis 0,1 Gew.-%, beispielsweise 0,01 Gew.-%, eingesetzt.

Für Farnesol ist beispielsweise eine Konzentration von 0,001 bis 1,5 Gew.-%, insbesondere von 0,01 bis 0,8 Gew.-% geeignet.

Die erfindungsgemäß verwendbaren Farnesol und die optionalen Monoterpene, Sesquiterpene und/oder Diterpene oder deren Derivate sind insbesondere zur Hemmung der asexuellen Vermehrung aller Pilze geeignet, die in den Stammlisten "DSMZ - List of Filamentous Fungi" und "DSMZ - List of Yeasts" der DSMZ (Deutsche Stammsammlung von Mikroorganismen und Zellkulturen GmbH, Braunschweig) aufgeführt sind. Die Listen sind im Internet unter der Adresse (http://www.dsmz.de/species/fungi.htm) bzw. (http://www.dsmz.de/species/yeasts.htm) einsehbar.

Bevorzugtermaßen sind die erfindungsgemäß verwendbaren Farnesol und die optionalen Monoterpene, Sesquiterpene und/oder Diterpene oder deren Derivate zur Hemmung der asexuellen Vermehrung von Pilzen geeignet. Dazu sind zum Beispiel die humanpathogenen Spezies der Klassen Ascomycota, Basidiomycota, Deuteromycota und Zygomycota zu zählen, insbesondere alle Spezies der Gattungen Aspergillus, Penicillium, Cladosporium und Mucor, sowie die humanpathogenen Formen von Candida.

Zu den Ascomycota gehören hier insbesondere alle Spezies der Gattungen Aspergillus, Penicillium und Cladosporium. Diese Pilze bilden Sporen aus, die bei Kontakt mit der Haut oder den Atemwegen ein stark allergieauslösendes Potential aufweisen. Zu den Basidiomycota ist beispielsweise Cryptococcus neoformans zu zählen. Zu den Deuteromycota sind alle als Schimmelpilze bekannten Gattungen zu zählen, insbesondere solche, die durch das Fehlen eines sexuellen Stadiums nicht den Klassen Ascomycota, Basidiomycota oder Zygomycota zugeordnet werden.

Die erfindungsgemäß verwendbaren Farnesol und die optionalen Monoterpene, Sesquiterpene und/oder Diterpene oder deren Derivate sind besonders bevorzugt zur Hemmung der Sporenbildung bei allen Spezies der Gattung Aspergillus geeignet, ganz besonders bevorzugt bei Spezies, die ausgewählt sind unter Aspergillus aculeatus, Aspergillus albus, Aspergillus alliaceus, Aspergillus asperescens, Aspergillus awamori, Aspergillus candidus, Aspergillus carbonarius, Aspergillus carneus, Aspergillus chevalieri, Aspergillus chevalieri var. intermedius, Aspergillus clavatus, Aspergillus ficuum, Aspergillus flavipes, Aspergillus flavus, Aspergillus foetidus, Aspergillus fumigatus, Aspergillus giganteus, Aspergillus humicola, Aspergillus intermedius, Aspergillus japonicus, Aspergillus nidulans, Aspergillus niger, Aspergillus niveus, Aspergillus ochraceus, Aspergillus oryzae, Aspergillus ostianus, Aspergillus parasiticus, Aspergillus parasiticus var. globosus, Aspergillus penicillioides, Aspergillus phoenicis, Aspergillus rugulosus, Aspergillus sclerotiorum, Aspergillus sojae var. gymnosardae, Aspergillus sydowi, Aspergillus tamarii, Aspergillus terreus, Aspergillus terricola, Aspergillus toxicarius, Aspergillus unguis, Aspergillus ustus, Aspergillus versicolor, Aspergillus vitricolae und Aspergillus wentii.

Gemäß einer besonders bevorzugten Ausführungsform sind Farnesol und die optionalen erfindungsgemäß verwendbaren Monoterpene, Sesquiterpene und/oder Diterpene oder deren Derivate ganz besonders bevorzugt zur Hemmung der Sporenbildung bei Spezies der Gattung Aspergillus, die ausgewählt sind unter *Aspergillus flavus* und *Aspergillus nidulans.*

Geeignete Gegenstände der vorliegenden Erfindung sind Baustoffe oder Bauhilfsstoffe, die zur Hemmung der asexuellen Vermehrung von Pilzen geeignetes Farnesol enthalten.

Die Ausrüstung der Baustoffe oder Bauhilfsstoffe erfolgt beispielsweise durch mechanisches Einarbeiten oder Aufbringen einer geeignet konzentrierten Lösung eines erfindungsgemäßen Mittels in bzw. auf die Baustoffe oder Bauhilfsstoffe.

Die erfindungsgemäß ausgerüsteten Baustoffe oder Bauhilfsstoffe sind ausgewählt unter Klebe- und Dichtungsmassen. Besonders bevorzugte Baustoffe oder Bauhilfsstoffe sind Fugendichtmassen (bspw. silikonhaltige Fugendichtmassen),Tapetenkleister, Putz, Teppichfixierer, Silikonkleber, Fliesenkleber.
Dichtungsmassen und insbesondere Fugendichtungsmassen enthalten typischerweise organische Polymere sowie in vielen Fällen mineralische oder organische Füllstoffe und sonstige Additive.
Geeignete Polymere sind beispielsweise thermoplastische Elastomere, wie in der DE-A-3602526 der Anmelderin beschrieben, vorzugsweise Polyurethane und Acrylate. Geeignete Polymere sind auch in den Offenlegungsschriften DE-A-3726547, DE-A-4029504 und DE-A-4009095 der Anmelderin sowie in DE-A-19704553 und DE-A-4233077 genannt, auf die hiermit in vollem Umfang Bezug genommen wird.
Die Dichtungsmassen und insbesondere Fugendichtungsmassen können wäßrige oder organische Lösungsmittel enthalten. Als organische Lösungsmittel kommen Kohlenwasserstoffe wie Cyclohexan, Toluol oder auch Xylol oder Petrolether in Frage. Weitere Lösungsmittel sind Ketone wie Methylbutylketon oder Chlorkohlenwasserstoffe.
Weiterhin können die Dichtungsmassen noch weitere kautschukartige Polymere enthalten. Hier kommen relativ niedermolekulare, handelsübliche Typen von Polyisobutylen, Polyisopren oder auch Polybutadienstyrol in Frage. Auch die Mitverwendung von abgebautem Naturkauschuk oder von Neoprenkautschuk ist möglich. Hier können auch bei Raumtemperatur noch fließfähige Typen eingesetzt werden, welche häufig als "Flüssigkautschuk" bezeichnet werden.
Die erfindungsgemäßen Dichtungsmassen können verwendet werden, um die verschiedensten Materialien miteinander zu verbinden bzw. abzudichten. Hier ist in erster Linie an die Verwendung auf Beton, auf Glas, auf Putz und/oder Emaille sowie Keramik und Porzellan gedacht. Aber auch das Verbinden bzw. Abdichten von Formteilen bzw. Profilen aus Aluminium, Stahl, Zink oder auch aus Kunststoffen wie PVC oder Polyurethanen oder Acrylharzen ist möglich. Schließlich sei das Abdichten von Holz oder Holzmaterialien mit den verschiedensten anderen Werkstoffen erwähnt.

Die Standfestigkeit von Fugendichtungsmassen wird in der Regel durch Zusatz von feinteiligen Feststoffen - auch Füllstoffe genannt - erzielt. Diese lassen sich in solche organischer und solche anorganischer Art unterscheiden. Als anorganische Füllstoffe können beispielsweise Kreide - gecoatet oder ungecoatet - und/oder Zeolithe bevorzugt sein. Letztere können zudem auch als Trockenmittel fungieren. Als organischer Füllstoff kommt z. B. PVC- Pulver in Betracht.
Die Füllstoffe tragen im allgemeinen wesentlich dazu bei, daß die Dichtungsmasse nach der Anwendung einen notwendigen inneren Halt besitzt, so daß ein Auslaufen oder Ausbuchten der Dichtungsmasse aus senkrechten Fugen verhindert wird. Die genannten Zusatz- bzw. Füllstoffe lassen sich in Pigmente und thixotropierende Füllstoffe, auch verkürzt als Thixotropiermittel bezeichnet, einteilen.
Als Thixotropierungsmittel eignen sich die bekannten Thixotropierungsmittel wir Bentone, Kaoline oder auch organische Verbindungen wie hydriertes Rizinusöl bzw. Derivate desselben mit mehrfunktionellen Aminen oder die Umsetzungsprodukte von Stearinsäure oder Rizinolsäure mit Ethylendiamin. Als besonders günstig hat sich die Mitverwendung von Kieselsäure, insbesondere von Kieselsäure aus der Pyrolyse erwiesen. Außerdem kommen als Thixotropiermittel im wesentlichen quellfähige Polymerpulver in Betracht. Beispiele sind hierfür Polyacrylnitril, Polyurethan, Polyvinylchlorid, Polyacrylsäureester, Polyvinylalkohole, Polyvinylacetate sowie die entsprechenden Copolymerisate. Besonders gute Ergebnisse lassen sich mit feinteiligem Polyvinylchloridpulver erhalten. Neben den Thixotropierungsmitteln können auch noch zusätzlich Haftvermittler eingesetzt werden wie etwa Mercaptoalkylsilan. Hier hat es sich als zweckmäßig erwiesen, ein Monomercaptoalkyltrialkoxysilan einzusetzen. Handelsüblich ist beispielsweise das Mercaptopropyltrimethoxysilan.

Die Eigenschaften einer Fugendichtungsmasse lassen sich noch weiter verbessern, wenn dem als Thixotropiermittel verwendeten Kunststoffpulver weitere Komponenten zugesetzt werden. Dabei handelt es sich um Stoffe, die unter die Kategorie der für Kunststoffe angewendeten Weichmacher bzw. Quellmittel und Quellhilfsmittel fallen. Es kommen z. B. Weichmacher aus der Klasse der Phthalsäureester in Betracht. Beispiele für anwendbare Verbindungen aus dieser Substanzklasse sind Dioctylphthalat, Dibutylphthalat und Benzylbutylphthalat. Weitere geeignete Substanzklassen sind Chlorparaffine, Alkylsulfonsäureester etwa der Phenole oder Kresole sowie Fettsäureester.

Als Quellhilfsmittel sind solche niedermolekularen organischen Substanzen einsetzbar, die mit dem Polymerpulver und dem Weichmacher mischbar sind. Derartige Quellhilfsmittel lassen sich den einschlägigen Kunststoff- und Polymer-Handbüchern für den Fachmann entnehmen. Als bevorzugte Quellhilfsmittel für Polyvinylchloridpulver dienen Ester, Ketone, aliphatische Kohlenwasserstoffe, aromatische Kohlenwasserstoffe sowie aromatische Kohlenwasserstoffe mit Alkylsubstituenten.

Als Pigmente und Farbstoffe werden die für diese Verwendungszwecke bekannten Substanzen wie Titandioxid, Eisenoxide und Ruß verwendet

Zur Verbesserung der Lagerstabilität werden bekanntermaßen den Dichtungsmassen Stabilisatoren wie Benzoylchlorid, Acetylchlorid, Toluolsulfonsäuremethylester, Carbodiimide und/oder Polycarbodiimide zugesetzt. Als besonders gute Stabilisatoren haben sich Olefine mit 8 bis 20 Kohlenstoffatomen erwiesen. Neben der stabilisierenden Wirkung können diese auch Aufgaben von Weichmachern bzw. Quellmitteln erfüllen. Bevorzugt werden Olefine mit 8 bis 18 Kohlenstoffatomen, insbesondere wenn die Doppelbindung in 1,2-Stellung angeordnet ist. Beste Ergebnisse erhält man, wenn die Molekülstruktur dieser Stabilisatoren linear ist.

Durch die erfindungsgemäße Verwendung von Farnesol zur Hemmung der asexuellen Vermehrung von Pilzen umgeht man das Problem der Resistenzbildung aufgrund biozider Wirkstoffe. Bei der Anwendung in schimmelgefährdeten Bau- und Bauhilfsstoffen, insbesondere in Dichtungsmassen und besonders bevorzugt in Fugendichtungsmassen werden durch die Hemmung der Sporenbildung mehrere erwünschte Effekte erzielt:
a) Verhinderung von Verfärbungen durch pigmentierte Sporen.
b) Verzögerung der Ausbreitung des Schimmelbefalls.
c) Verminderung der Allergenbelastung.

Eine weitere bevorzugte Ausführungsform der vorliegenden Erfindung sind Tapetenkleber, enthaltend 0,001 bis 1,5 Gew.-% zur Hemmung der asexuellen Vermehrung von Pilzen geeignete Farnesol. Tapetenkleister aus wässrigen Lösungen von Hydrokolloiden wie Methylcellulose, Methylhydroxypropylcellulose oder wasserlöslichen Stärkederivaten. Auch wässrige Dispersionen von filmbildenden Hochmolekularen wie Polyvinylacetat können, insbesondere in Verbindung mit den bereits erwähnten Cellulose- und Stärkederivaten, eingesetzt werden.

Die Konzentration des Farnesols zur Hemmung der asexuellen Vermehrung von Pilzen in den erfindungsgemäßen Mitteln kann durch den Fachmann in einem breiten Bereich von 0,001 bis 1,5 Gew.-% variiert werden, abhängig von den Einsatzbedingungen der Mittel.

Die erfindungsgemäßen Mittel werden nach üblichen und dem Fachmann bekannten Rezepturen hergestellt. Das zur Hemmung der asexuellen Vermehrung von Pilzen geeignete Farnesol wird vorzugsweise den bereits fertig zubereiteten Mitteln zugegeben, sie können aber auch während des Herstellungsprozesses zugesetzt werden, wenn dies gewünscht ist.

Die Hemmung der asexuellen Vermehrung von Pilzen auf Textilien oder Kunststoffoberflächen verhindert häufig eine Reinfektion von zuvor bereits befallenen Körperbereichen. Die Hemmung der asexuellen Vermehrung von Pilzen auf Keramiken, Kunststoffen oder Metallen verringert das Infektions- bzw. Reinfektionsrisiko, ohne die Haut, die Schleimhäute oder die Abwässer mit fungizid bzw. fungistatisch wirkenden Substanzen zu belasten. Ebenso können Katheter sowie andere aus Kunststoff oder Metallen hergestellte medizinische Geräte und/oder Prothesen durch die Verwendung zur Hemmung der asexuellen Vermehrung von Pilzen geeigneter Monoterpene, Sesquiterpene und/oder Diterpene oder deren Derivate beispielsweise in Spülungen oder Reinigungsmitteln weitgehend von Pilzen freigehalten werden. Die folgenden Beispiel sollen die Erfindung erläutern, ohne sie jedoch darauf einzuschränken.

### Beispiele:

### Beispiel 1

### Auswirkung von Farnesol auf die Sporenbildung bei Aspergillus niger

Je 100 µl einer Zellsuspension von Aspergillus niger (1,5 * 10⁷ Zellen/ml) wurden auf Malzagarextrakt (Meck) ausplattiert, denen zuvor unterschiedliche Mengen an Farnesol beigefügt worden waren, nämlich 0; 25; 62,5; 125; 250 und 500 µM. Die Platten wurden 5 Tage bei 25 °C inkubiert und die Sporenbildung wurde anschließend per Augenschein überprüft und die Sporulationshemmung abgeschätzt (vgl. Tab. 1). Alle eingesetzten Konzentrationen von Farnesol behinderten das Wachstum nicht, die Sporenbildung wurde jedoch mit steigenden Konzentrationen gehemmt und bei 500 µM vollständig unterdrückt.

**Tab.1.: Sporulation bei verschiedenen Konzentrationen von Farnesol**

| | | | | | | |
|---|---|---|---|---|---|---|
| Konzentration Farnesol [µM] | 0 | 25 | 62,5 | 125 | 250 | 500 |
| Sporulation [%] | 100 | 90 | 75 | 50 | 10 | 0 |

### Beispiele 2 bis 4: Tapetenklebstoffe

### Beispiel 2

| **Inhaltsstoffe** | **Menge** |
|---|---|
| Methylcellulose (300 m • Pas in 2 %iger wässriger Lösung, Methoxylgehalt 26 %) | 500 g |
| PVAcetat-Redispersionspulver | 350 g |
| Kaolin | 60 g |
| Cellulosepulver | 50 g |
| Additionsprodukt von 6 Mol Ethylenoxid an 1 Mol Nonylphenol | 10 g |
| handelsüblicher Konservierungsstoff (Basis Isothiazolinderivat) | 8 g |
| Farnesol | 0,2 g |

### Beispiel 3

| **Inhaltsstoffe** | **Menge** |
|---|---|
| Methylcellulose (5000 m • Pas in 2 % wässrige Lösung, Methoxylgehalt 19 %) | 680 g |
| Carboxylmethylstärke (DS 0,22) | 300 g |
| Additionsprodukt von 4 Mol Ethylenoxid an 1 Mol Fettalkohol | 15 g |
| handelsüblicher Konservierungsstoff (Basis Isothialzolinderivat) | 10 g |
| Farnesol | 0,2 g |

### Beispiel 4

| **Inhaltsstoffe** | **Menge** |
|---|---|
| handelsübliche Polyvinylacetatdisper-sionenen (50 % Feststoffgehalt) | 500 g |
| Wasser | 200 g |
| Methylcellulose (3000 m • Pas in 2 % wässriger Lösung) | 20 g |
| handelsübliches Konservierungsmittel | 10 g |
| Farnesol | 0,15 g |

Die erhaltenen Mischungen werden mit Wasser im Verhältnis 1:20 (2) oder 1:25 (3) oder 1:1 (4) angerührt und zum Aufkleben von handelsüblichen Tapeten auf Wandflächen benutzt.

### Beispiel 5: Flüssigwaschmittel (Referenzbeispiel)

| **Rohstoff** | Menge in Gewichtsprozent |
|---|---|
| C₁₂-C₁₈ Fettalkohol + 7 EO (Dehydol LT 7, Cognis) | 15 |
| C₁₂-C₁₄ Fettalkohol + 2 EO-sulfat, Natriumsalz (Texapon N 70, Cognis) | 7 |
| C₈₋₁₈-Fettsäure geschnitten (Kokosölfettsäure, Edenor K12-18, Cognis) | 8 |
| Natriumcitrat | 1,5 |
| Enzyme | + |
| Farbstoff | + |
| Parfüm | + |
| Farnesol | 0,4 |
| Wasser | Ad 100 |

## Patentansprüche

1. Klebstoff, enthaltend 0,001 bis 1,5 Gew.-% Farnesol.

2. Klebstoff nach Anspruch 1, **dadurch gekennzeichnet, dass** es sich um einen Klebstoff auf Wasserbasis handelt.

3. Klebstoff nach Anspruch 1 oder 2, **dadurch gekennzeichnet, dass** es sich um einen Klebstoff zur Befestigung von Tapeten und ähnlichen Wandbelagsstoffen handelt.

4. Dichtungsmasse, enthaltend 0,001 bis 1,5 Gew.-% Farnesol.

5. Dichtungsmasse gemäß Anspruch 4, **dadurch gekennzeichnet, dass** es sich um eine Fugendichtmasse handelt.

## Claims

1. Adhesive containing from 0.001 to 1.5 wt.% farnesol.

2. Adhesive according to claim 1, **characterized in that** the adhesive is a water-based adhesive.

3. Adhesive according to claim 1 or 2, **characterized in that** the adhesive is an adhesive for attaching wallpaper and similar wall covering materials.

4. Sealant containing from 0.001 to 1.5 wt.% farnesol.

5. Sealant according to claim 4, **characterized in that** the sealing compound is a joint sealant.

## Revendications

1. Adhésif contenant 0,001 à 1,5% en poids de farnésol.

2. Adhésif selon la revendication 1, **caractérisé en ce qu'**il est un adhésif à base d'eau.

3. Adhésif selon la revendication 1 ou 2, **caractérisé en ce qu'**il est un adhésif destiné à la fixation de papiers peints et de revêtements muraux similaires.

4. Matière d'étanchéité contenant de 0,001 à 1,5% en poids de farnésol.

5. Matière d'étanchéité selon la revendication 4, **caractérisée en ce qu'**elle est une matière d'étanchéité pour joints.
